# EUROPEAN PATENT APPLICATION

(11) **EP 3 192 566 A1**
(43) Date of publication of application: **19.07.2017**
(21) Application number: 17151665.1
(22) Date of filing: 16.01.2017
(51) Int. Cl.: A61Q 13/00, A61K 8/86, A61K 8/41, C11B 9/00, C11D 1/00, C11D 1/44, C11D 3/50

(54) **POLYALKOXY-POLYIMINE ADDUCTS FOR USE IN DELAYED RELEASE OF FRAGRANCE INGREDIENTS**

(30) Priority: 15.01.2016 US 201662279387 P; 13.01.2017 US 201715405595
(71) Applicant: International Flavors & Fragrances Inc., New York, NY 10019 (US)
(72) Inventor: VELIATH, Elizabeth Dharma, Ewing, NJ New Jersey 08638 (US); CHERKAUSKAS, John, Burlington, NJ New Jersey 08016 (US); MONTELEONE, Michael G., Hazlet, NJ New Jersey 07730 (US); SCHIET, Franc T., 1412 GC Naarden (NL); CARIOU, Pierre Yves, 92200 Neuilly-sur-Seine (FR); PLUYTER, Johan Gerwin Lodewijk, Middletown, NJ New Jersey 07748 (US); SASAKI, Takashi, Matawan, NJ New Jersey 07747 (US)
(74) Representative: Lawrence, John

(57) **Abstract**

Disclosed is a fragrance adduct that is a reaction product between a polymeric amine and a reactive fragrance ingredient. The polymeric amine contains a backbone and at least one amino functional group, the reactive fragrance ingredient is an aldehyde, a ketone, an α,β-unsaturated electrophilic compound reactive towards the polymeric amine, or a combination thereof, and the reaction product is an imine compound or a Michael addition adduct. The fragrance adduct can be formulated into a fragrance composition, household product, personal care product, hair care product, or beauty care product. Also disclosed is a method for sequestering a fragrance using the fragrance adduct.

## Description

### BACKGROUND

The delayed and sustained release of fragrance ingredients is a sought-after property to extend the benefit of fragrance in many consumer applications. Typically neat fragrance oils have a limited performance duration due to the volatile nature of the materials. Encapsulation technologies seek to entrap the fragrance ingredients inside supramolecular structures; however some of these technologies are limited to use of fragrance ingredients that will not react with the encapsulation components themselves, *e.g.,* free amine groups in polyurea capsules.

Approaches for cross-linking fragrances to gel polymers, copolymerization or trapping fragrances within polymeric matrices have been described. See, US 7,700,665, WO 2012/071261, and WO 2010/094356. Further, co-delivery of fragrances with polyoxyalkylene amines has been suggested. See WO 2009/065738. In addition, the use of a modified amino-functional polymer or polyvinylamine in the production of a pro-fragrance compound has been described. See WO 2009/153209 and US 2006/0204462.

### SUMMARY OF THE INVENTION

One aspect of this invention relates to a fragrance adduct that is a reaction product between a polymeric amine and a reactive fragrance ingredient, in which the polymeric amine has a molecular weight of 100 to 10,000,000 Daltons (e.g., 100 to 10000 and 200 to 5000) and contains a backbone and at least one amino functional group; the reactive fragrance ingredient is an aldehyde, a ketone, an α,β-unsaturated electrophilic compound reactive towards the polymeric amine, or a combination thereof; and the reaction product is an imine compound or a Michael addition adduct.

In the polymeric amine, the backbone can be polyalkylene or polyoxyalkyleneamine. Example of the polymeric amine includes polyoxyalkyleneamine containing propylene oxide, ethylene oxide, butylene oxide or a mixture thereof, and the following compounds: 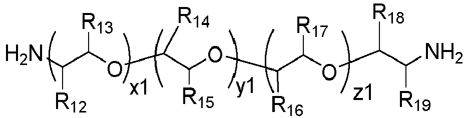 y1 being 1 to 50 and x1 + z1 being 0 to 100
each of R₁₂, R₁₃₅ R₁₄, R₁₅₅ R₁₆, R₁₇, R₁₈, and R₁₉, independently, being H,

CH₃, or CH₂CH₃

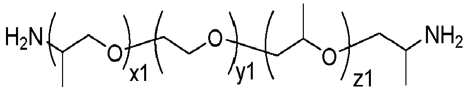

y1 being 1 to 50 and x1 + z1 being 0 to 100 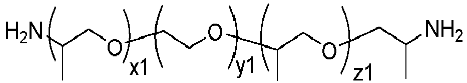 y1 being 1 to 50 and x1 + z1 being 0 to 100 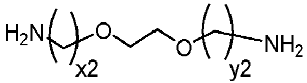 x2 being 0 to 50 and y2 being 0 to 50 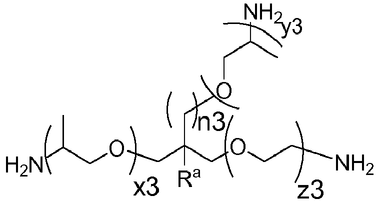 x3+y3+z3 being 1 to 200, n3 being 0 to 10, and R^{a} being H, CH₃, or C₂H₅ 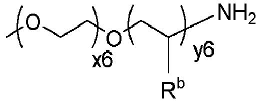 x6 being 1 to 80, y6 being 1 to 80, and R^{b} being H, CH₃, or CH₂CH₃ 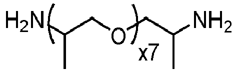 x7 being 1 to 80 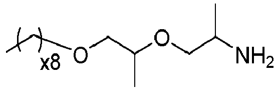 x8 being 4 to 24 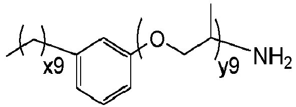 x9 being 1 to 18 and y9 being 5 to 25 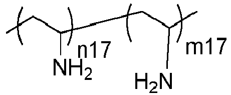 each of m17 and n17, independently, being 300 to 40,000 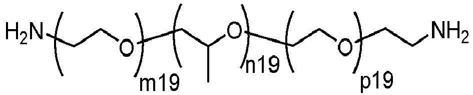 each of m19, n19, and p19, independently, being 1 to 100 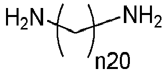 n20 being 6 to 24 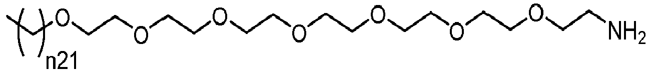 n21 being 10 to 50 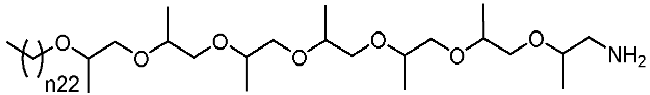 n22 being 14 to 50 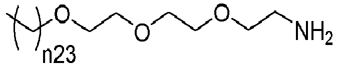 n23 being 10 to 50 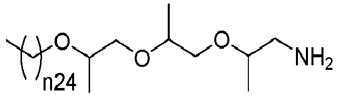 n24 being 10 to 50 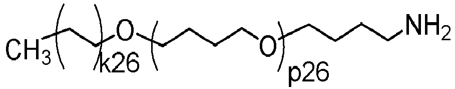 k26 being 4 to 25 and p26 being 2 to 20 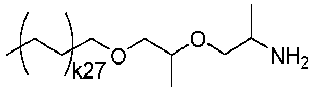 k27 being 2 to 24

In preferred embodiments, the polymeric amine is 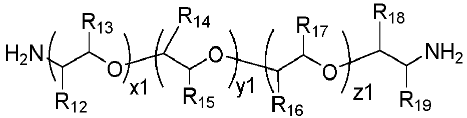 y1 being 3 to 20 and x1 + z1 being 20 to 50
each of R₁₂, R₁₃, R₁₄, R₁₅, R₁₆, R₁₇, R₁₈, and R₁₉, independently, being H,

CH₃, or CH₂CH₃

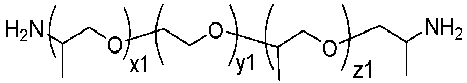

y1 being 3 to 20 and x1 + z1 being 20 to 50 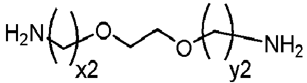 x2 being 2 to 12 and y2 being 2 to 12 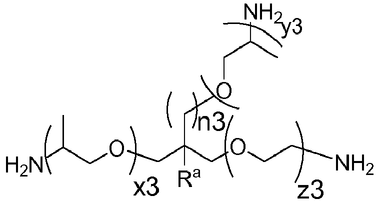 x3+y3+z3 being 50 to 120, n3 being 0 to 4, and R^{a} being H, or CH₃ 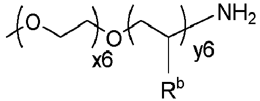 x6 being 4 to 50, y6 being 4 to 50, and R^{b} being H, or CH₃
or 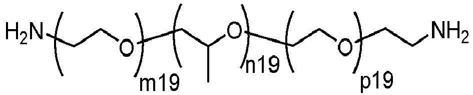 each of m19, n19, and p19, independently, being 4 to 40.

Turning to the reactive fragrance ingredient, it is a aldehyde, a ketone, an α,β-unsaturated carbonyl compound, an α,β-unsaturated nitrile, an α,β-unsaturated nitroxide, or a combination thereof These compounds are reactive towards the polymeric amines. In some embodiments, the fragrance adduct is an imine formed between the polymeric amines and the reactive fragrance ingredient selected from the group consisting of aldehydes, ketones, and combinations thereof. In other embodiments, the fragrance adduct is a Michael addition adduct formed between the polymeric amine and the fragrance ingredient selected from the group consisting of α,β-unsaturated carbonyl compounds, α,β-unsaturated nitriles, α,β-unsaturated nitroxides, and combinations thereof

Examples of the reactive fragrance ingredient is 6-decenal, 3-(4-isobutylcyclohexyl)propanal, 2-octahydro-1H-4,7-methanoinden-5-yl)acetaldehyde, 2-methylpentan-3-yl (E)-but-2-enoate, 4-(heptyloxy)-3-methylbutanal, 7,7,8,9,9-pentamethyl-6,7,8,9-tetrahydro-5H-cyclopenta[h]quinazoline, or a combination thereof.

Another aspect of this invention relates to a fragrance composition comprising the fragrance adduct described above.

Also within the scope of this invention is a household product, personal care product, hair care product or beauty care product comprising the fragrance adduct described above.

Still within the scope of this invention is a method for sequestering a fragrance comprising binding a fragrance to a polymeric amine to form a fragrance adduct thereby sequestering the fragrance, wherein the fragrance is a an aldehyde, a ketone, an α,β-unsaturated electrophilic compound reactive towards the polymeric amine, or a combination thereof. The fragrance adduct can react with a malodor acid or malodor thiol compound thereby releasing the fragrance from the fragrance adduct.

The details of one or more embodiments of the invention are set forth in the description below. Other features, objects, and advantages of the invention will be apparent from the description and the claims.

### DETAILED DESCRIPTION OF THE INVENTION

Aldehydes are generally not suitable for use in conventional polyurea capsule delivery systems due to reactivity with the wall forming components of the system. It has now been found that a polymeric di-, tri- or polyamine, generally referred to herein as an "polymer," can be reacted with a fragrance ingredient that is an aldehyde, ketone, or α,β-unsaturated electrophilic compound (e.g., an alpha-beta unsaturated carbonyl compound) to sequester the fragrance ingredient.

More specifically, one or more free amine functional groups of the polymer react with either a carbonyl of the fragrance to form an imine (Scheme 1), or an alpha-beta unsaturated carbonyl compound to form a Michael addition adduct (Scheme 2) thereby forming the fragrance adduct.

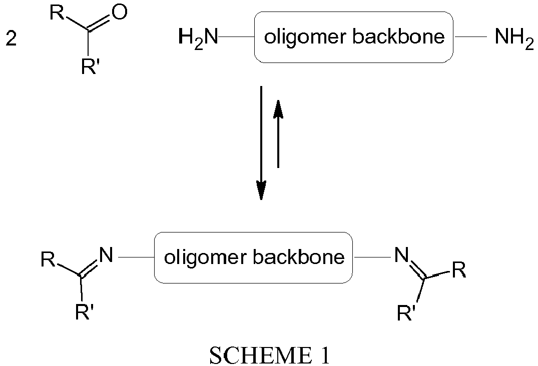

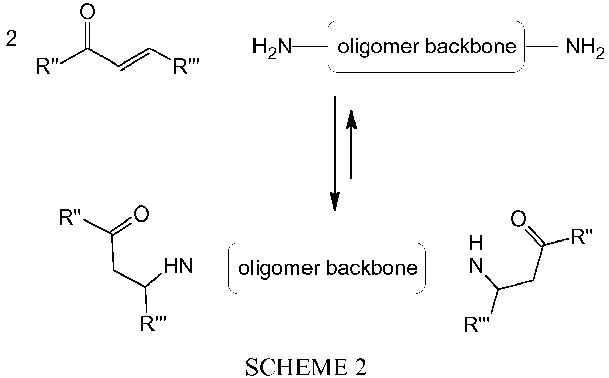

The polymer can be mixed with either a single fragrance ingredient first, or a mixture of materials containing a plurality of different fragrance ingredients. The resulting imine or Michael addition adduct composition can then be used in a fragrance accord as a delayed delivery system, which slowly releases the sequestered fragrance ingredient under mildly acidic conditions with water or moisture to provide lasting fragrance performance as well as malodor control.

Accordingly, the present invention provides fragrance adducts and delivery compositions and methods for sequestering fragrance ingredients using a polymeric amine. In particular, the fragrance adduct of the invention is an imine or Michael adduct formed between a reactive fragrance ingredient and a polymeric amine. The reactive fragrance ingredient is selected from the group consisting of aldehydes, ketones, an alpha,beta-unsaturated electrophilic compounds (e.g., alpha,beta-unsaturated carbonyl compounds).

As used herein, the tern "polymer" as used herein refers to a compound having two or more repetitive units. As such, polymers also include oligomers, which have two to ten repetitive units.

Water soluble diamines are one class of polymeric amines of use in this invention such as:

H₂N(CH₂)ₙNH₂,

where n is > 1 *(e.g.,* 2 to 1,000,000 and 10 to 500,000). Exemplary amines of this type include, but are not limited to, 1,3-diaminopropane, 1,4-diaminobutane, hexanethylene diamine, hexamethylene diamine, and pentaethylenehexamine.

Polymeric amines that have a functionality greater than 2 include polyalkylene polyamines of the type: 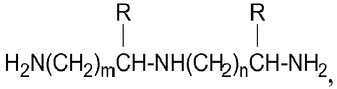 where R is hydrogen or -CH₃, m is 1-5 and n is 1-5, *e.g.,* diethylene triamine, triethylene tetraamine and the like. Exemplary amines of this type include, but are not limited to diethylenetriamine, bis(3-aminopropyl)amine, bis(hexanethylene)triamine.

In particular embodiments, the polymeric amine is a polyoxyalkyleneamine. Polyoxyalkyleneamines include two of more primary or secondary amino groups attached to a backbone, derived, for example, from propylene oxide, ethylene oxide, butylene oxide or a mixture thereof. The ether amine can be monoamine, diamine, or triamine, based on this core structure. An example is:

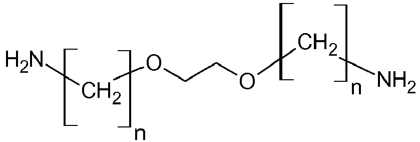

Exemplary polyoxyalkyleneamines include 2,2'-ethylenedioxy)bis (ethylamine) and 4,7,10-trioxa-1,13-tridecanediamine, as well as those available under the designation JEFFAMINE, such as, without limitation, JEFFAMINE D-230, D-400, D-2000, HK-511, ED-600, ED-900, ED-2003, T-403, T-3000, T-5000, SD-231, SD-401, SD-2001, and ST-404 (Huntsman Corporation). Such amines have an approximate molecular weight ranging from 200 to 7500.

Other suitable amines include, but are not limited to, tris(2-aminoethyl)amine, triethylenetetramine, N,N'-bis(3-aminopropyl)-1,3-propanediamine, tetraethylene pentamine, 1,2-diaminopropane, N,N,N',N'-tetrakis(2-hydroxyethyl)ethylene diamine, N,N,N',N'-tetrakis(2-hydroxypropyl)ethylene diamine, branched polyethylenimine, 2,4-diamino-6-hydroxypyrimidine and 2,4,6-triaminopyrimidine.

Fragrances that can be sequestered with the polymers described herein include, but are not limited to, individual fragrances, any combination of fragrance oils, essential oils, plant extracts or mixture thereof, which contain an aldehyde, ketone, or an alpha-beta unsaturated carbonyl functional group. Exemplary ketones include, but are not limited to buchu oxime; isojasmone; methyl-β-naphthyl ketone, musk indanone; tonalide/musk plus; α-damascone, β-damascone, δ-damascone, isodamascone, damascenone, damarose, methyl dihydrojasmonate, menthone, carvone, camphor, fenchone, α-ionene, β-ionone, dihydro-β-ionone, γ-methyl so-called ionone, fleuramone, dihydrojasmone, cis-jasmone, iso-E-super, methyl cedrenyl ketone or methyl cedrylone, acetophenone, methyl acetophenone, paramethoxyacetophenone, methyl-β-naphthyl ketone, benzylacetone, benzophenone, parahydroxy-phenylbutanone, celery ketone or livescone, 6-isopropyl- decahydro-2-naphthone, dimethyloctenone, freskomenth, 4-(1-ethoxyvinyl)-3,3,5,5-tetramethylcyclohexanone, methylheptenone, 2-(2-(4-methyl-3-cyclohexen-1- yl)propyl)cyclopentanone, 1-(p-menthen-6(2)-yl)-1-propanone, 4-(4-hydroxy-3-methoxyphenyl)-2-butanone, 2-acetyl-3,3-dimethylnorbornane, 6,7-dihydro-1,1,2,3,3-pentamethyl-4(5H)indanone, 4-damascole, dulcinyl or cassion, gelsone, hexalone isocyclemon E, methyl cyclocitron, methyl lavendel ketone, orivone, para-*tert*-butylcyclohexanone, verdone, delphone, muscone, neobutenone, plicatone, veloutone, 2,4,4,7-tetramethyloct-6-en-3-one, tetramerane, hedione, and mixtures thereof. The ketones may preferably be selected from α-damascone, δ-damascone isodamascone, carvone, γ-methylionone, iso-E-super, 2,4,4,7-tetramethyloct-6-en-3-one, benzylacetone, β-damascone, damascenone, methyl dihydrojasmonate, methyl cedrylone, hedione and mixtures thereof.

Suitable fragrances containing aldehydes include individual aldehydes or aldehyde mixtures such as, for example, melonal, triplal, ligustral, adoxal, anisaldehyde, cymal, ethyl vanillin, florhydral, helional, heliotropin, hydroxycitronellal, koavone, lauryl aldehyde, lyral, methyl nonylacetaldehyde, p,t-bucinal, phenyl acetaldehyde, undecylene aldehyde, vanillin, 2,6,10-trimethyl-9-undecenal, 3-dodecen-1-al, α-n-amylcinnamaldehyde, 4-methoxybenzaldhyde, benzaldehyde, 3-(4-tert-butylphenyl)propanal, 2-methyl-3-(para-methoxyphenylpropanal), 2-methyl-4-(2,6,6trimethyl-2(1)-cyclohexen-1-yl)butanal, 3-phenyl-2-propenal, cis-/trans-3,7-dimethyl-2,6-octadien-1-al, 3,7-dimethyl-6-octen-1-al, [(3,7-dimethyl-6octenyl)oxy]acetaldehyde, 4-isopropylbenzaldehyde, 1,2,3,4,5,6,7,8-octahydro-8,8-dimethyl-2-naphthaldehyde, 2,4-dimethyl-3-cyclohexen-i-carboxyaldehyde, decylaldehyde, 2,6-dimethyl-5-heptenal; 4-(tricyclo(5.2.1.0(2,6)]decylidene-8)butanal, octahydro-4,7-methano-IH-indenecarboxaldehyde, 3-ethoxy-4-hydroxybenzaldehyde, para-ethyl-α,α-dimethylhydrocinnamaldehyde, α-methyl-3,4(methylenedioxy)hydrocinnamaldehyde, 3,4-methylenedioxybenzaldehyde, α-n-hexylcinnamaldehyde, m-cymene-7-carboxaldehyde, α-methyl-phenylacetaldehyde, 7-hydroxy-3,7-dimethyloctanal, undecenal, 2,4,6-trimethyl-3-cyclohexene-1-carboxaldehyde, 4-(3)(4-methyl-3-pentenyl)-3 cyclohexene carboxaldehyde, 1-dodecanal, 2,4-dimethylcyclohexene-3-carboxaldehyde, 4-(4-hydroxy-4-methylpentyl)-3-cyclohexene-1-carbox-aldehyde, 7-methoxy-3,7-dimethyloctan-1-al, 2-methylundecanal, 2-methyldecanal, 1-nonanal, 1-octanal, 2,6,10-trimethyl-5,9-undecadienal, 2-methyl-3-(4-tert-butyl)propanal, dihydrocinnamaldehyde, 1-methyl-4-(4-methyl-3-pentenyl)-3-cyclohexene-1-carboxaldehyde, 5- or 6- methoxyhexahydro-4,7-methanoindan-1- or 2-carboxyaldehyde, 3,7-dimethyloctan-1-al, 1-undecanal, 10-undecen-1-al, 4-hydroxy-3methoxybenzaldehyde, 1-methyl-3 -(4-methylpentyl)-3 - cyclohexenecarboxyaldehyde, 7-hydroxy-3,7dimethyloctanal, trans-4-decenal, 2,6-nonadienal, para-tolylacetaldehyde, 4-methylphenylacetaldehyde, 2-methyl-4-(2,6,6-trimethyl-1-cyclohexen-1-yl)-2-butenal, ortho-methoxycinnamaldehyde, 3,7-dimethyl-2-methylene-6-octenal, phenoxyacetaldehyde, 5,9-dimethyl-4,8-decadienal, peony aldehyde (6,10-dimethyl-3-oxa-5,9-undecadien-1-al), hexahydro-4,7-methanoindane-1-carboxaldehyde, 2-methyloctanal, a-methyl-4-(1-nnethylethyl)benzeneacetaldehyde, 6,6-dimethyl-2-norpinene-2-propionaldehyde, para-methylphenoxyacetaldehyde, 2-methyl-3-phenyl-2-propen-1-al, 3,5,5-trimethylhexanal, hexahydro-8,8-dimethyl-2-naphthaldehyde, 3-propylbicyclo[2.2.1]-hept-5-ene-2-carbaldehyde, 9-decenal, 3-methyl-5-phenyl-1-pentanal, methylnonylacetaldehyde, 1-p-menthene-q-carboxaldehyde, citral or mixtures thereof, lilial citral, 1-decanal, florhydral, or 2,4-dimethyl-3-cyclohexene-i-carboxaldehyde. Preferred aldehydes may be selected from cis/trans-3,7-dimethyl-2,6octadien-l-al, heliotropin, 2,4,6-trimethyl-3-cyclohexene-1-carboxaldehyde, 2,6-nonadienal, α-n-amylcinnamaldehyde, α-n-hexylcinnamaldehyde, p-tert-bucinal, lyral, cymal, methyl-nonylacetaldehyde, trans-2-nonenal, lilial, trans-2-nonenal and mixtures thereof

In particular embodiments, the fragrance is 6-decenal (Opalene), 3-(4-isobutylcyclohexyl)propanal (Starfleur), 2-octahydro-1H-4,7-methanoinden-5-yl)acetaldehyde (Aquaflora), 2-methylpentan-3-yl (E)-but-2-enoate (Cosmofruit), 7,7,8,9,9-pentamethyl-6,6a,7,8,9,9a-hexahydro-5H-cyclopenta[h]quinazoline (Ambertonic), 4-(heptyloxy)-3-methylbutanal (Cristalfizz), 7,7,8,9,9-pentamethyl-6,7,8,9-tetrahydro-5H-cyclopenta[h]quinazoline (Sinfonide) 2-[(4-methylphenyl)methylene]-heptanal (Acalea), iso-amyl oxyacetic acid allylester (Allyl Amyl Glycolate), (3,3-dimethylcyclohexyl)ethyl ethyl propane-1,3-dioate (Applelide), (E/Z)-1-ethoxy-1-decene (Arctical), 2-ethyl-4-(2,2,3-trimethyl-3-cyclo-penten-1-yl)-2-buten-1-ol (Bacdanol), 2-methyl-3-[(1,7,7-trimethylbicyclo[2.2.1]hept-2-yl)oxy] exo-1-propanol (Bornafix), 1,2,3,5,6,7-hexahydro-1,1,2,3,3-pentamethyl-4H-inden-4-one (Cashmeran), trimethylcyclopentenylmethyloxabicyclooctane (Cassiffix), 1,1-dimethoxy-3,7-dimethyl-2,6-octadiene (Citral DMA), 3,7-dimethyl-6-octen-1-ol (Citronellol), 3A,4,5,6,7,7A-hexahydro-4,7-methano-1H-inden-5/6-yl acetate (Cyclacet), 3A,4,5,6,7,7A-hexahydro-4,7-methano-1H-inden-5/6-yl propinoate (Cyclaprop), 3A,4,5,6,7,7A-hexahydro-4,7-methano-1G-inden-5/6-yl butyrate (Cyclobutanate), 1-(2,6,6-trimethyl-3-cyclohexen-1-yl)-2-buten-1-one (Delta Damascone), 3-(4-ethylphenyl)-2,2-dimethyl propanenitrile (Fleuranil), 3-(O/P-ethylphenyl) 2,2-dimethyl propionaldehyde (Floralozone), tetrahydro-4-methyl-2-(2-methylpropyl)-2H-pyran-4-ol (Floriffol), 1,3,4,6,7,8-hexahydro-4,6,6,7,8,8-hexamethylcyclopenta-gamma-2-benzopyran (Galaxolide), 1-(5,5-dimethyl-1-cyclohexen-1-yl)pent-4-en-1-one (Galbascone), E/Z-3,7-dimethyl-2,6-octadien-1-yl acetate (Geranyl Acetate), α-methyl-1,3-benzodioxole-5-propanal (Helional), 1-(2,6,6-trimethyl-2-cyclohexen-1-yl)-1,6-heptadien-3-one (Hexalon), (Z)-3-hexenyl-2-hydroxybenzoate (Hexenyl Salicylate, CIS-3), 4-(2,6,6-trimethyl-2-cyclohexen-1-yl)-3-buten-2-one (Ionone α), 1-(1,2,3,4,5,6,7,8-octahydro-2,3,8,8-tetramethyl-2-naphthalenyl)-ethan-1-one (Iso E Super), methyl 3-oxo-2-pentylcyclopentaneacetate (Kharismal), 2,2,4-trimethyl-4-phenyl-butanenitrile (Khusinil), 3,4,5,6,6-pentamethylhept-3-en-2-one (Koavone), 3/4-(4-hydroxy-4-methyl-pentyl) cyclohexene-1-carboxaldehyde (Lyral), 3-methyl-4-(2,6,6-trimethyl-2-cyclohexen-1-yl)-3-buten-2-one (Methyl Ionone γ), 1-(2,6,6-trimethyl-2-cyclohexen-1-yl) pent-1-en-3-one (Methyl Ionone α Extra, Methyl Ionone N), 3-methyl-4-phenylbutan-2-ol (Muguesia), cyclopentadec-4-en-1-one (Musk Z4), 3,3,4,5,5-pentamethyl-11,13-dioxatricyclo[7.4.0.0<2,6>]tridec-2(6)-ene (Nebulone), 3,7-dimethyl-2,6-octadien-1-yl acetate (Neryl Acetate), 3,7-dimethyl-1,3,6-octatriene (Ocimene), ortho-tolylethanol (Peomosa), 3-methyl-5-phenylpentanol (Phenoxanol), 1-methyl-4-(4-methyl-3-pentenyl) cyclohex-3-ene-1-carboxaldehyde (Precyclemone B), 4-methyl-8-methylene-2-adamantanol (Prismantol), 2-ethyl-4-(2,2,3-trimethyl-3-cyclopenten-1-yl)-2-buten-1-ol (Sanjinol), 2-methyl-4-(2,2,3-trimethyl-3-cyclopenten-1-yl)-2-buten-1-ol (Santaliff), Terpineol, 2,4-dimethyl-3-cyclohexene-1-carboxaldehyde (Triplal), decahydro-2,6,6,7,8,8-hexamethyl-2H-indeno[4,5-B]furan (Trisamber), 2-tert-butylcyclohexyl acetate (Verdox), 4-tert-butylcyclohexyL acetate (Vertenex), acetyl cedrene (Vertofix), 3,6/4,6-dimethylcyclohex-3-ene-1-carboxaldehyde (Vertoliff), and (3Z)-1-[(2-methyl-2-propenyl)oxy]-3-hexene (Vivaldie) or a mixtures thereof.

The imine or Michael adduct materials of the invention can be combined with each other and/or other malodor counteractant compounds, e.g., as described in US 7,993,633; US 2013/0101544; US 2013/0101545 and US 2012/0294821.

The fragrance adduct and composition of the invention is of particular use in a method for sequestering a fragrance. In accordance with such a method, a polymeric amine and a reactive fragrance ingredient are reacted to form a fragrance adduct such as an imine compound or a Michael addition adduct. The fragrance adduct comprises a polymeric amine moiety and a reactive fragrance moiety. The two moieties are covalently bound to sequester the fragrance without the need for encapsulation. This method can be used to delay release of high impact aldehyde materials, which are reactive to the encapsulating materials. As such encapsulating these materials have not been previously available.

The fragrance adduct is then used in a fragrance accord as a delayed delivery system. Imines and Michael addition adducts are known to hydrolyze slowly in the presence of an acid and moisture (*i.e.,* water), triggering the release of the sequestered fragrance ingredient.

Other unreactive fragrance ingredients can be adsorbed to the backbone of the polymeric amine or fragrance adduct via non-covalent attractive forces (H-bonding, etc) which alters the materials volatility. As such, in some embodiments, the fragrance adduct further comprises a second (third, fourth, fifth, or sixth) fragrance ingredient binding to the fragrance adduct via a non-covalent attractive force. This is an additional mechanism of delayed fragrance ingredient release. Taken altogether, the system has both chemically bonded and non- chemically bonded fragrance ingredients that have delayed fragrance release and malodor control.

Malodor acids, such as butyric and isovaleric, can be such acidic triggers to cause the hydrolysis and release of the fragrance ingredient. The resulting free amine is able to neutralize the malodor acid in an acid-base ion pair.

Moreover, in addition to the polymeric amine freed from the hydrolysis, unreacted amino groups of the polymeric amine can also neutralize a malodor acid or malodor thiol compound in a product. For the purposes of the present invention, a compound neutralizes a malodor if it measurably (either qualitatively or quantitatively) reduces the perception or intensity of a malodor. In particular embodiments, the polymeric amine reduces the perception or intensity of a malodor by 50-100% as compared to the malodor in the absence of the polymeric amine. When the polymeric amine is used in combination with a fragrance, the fragrance can result in a further reduction in the perception or intensity of a malodor. In particular embodiments, a polymeric amine of the invention reduces the perception or intensity of a malodor by at least 50, 55, 60, 65, 70, 75, 80, 85, 90, 95 or 100% as compared to the malodor in the absence of the polymeric amine.

The inventive imine or Michael adduct composition can be included in any fragrance oil or accord wherever such an application calls for such a use. Exemplary products include, but are not limited to, household products (laundry detergents, scent boosters, fabric conditioners, dish detergent, AP cleaners, room deodorizers, candles, etc); personal care products (bar soap/wash/gel, sunscreen, antiperspirant/deodorant, lotions, powders, toiletries, shave cream, etc); hair care products (shampoo, conditioner, rinses, styling products, etc); and beauty care (fine fragrance, solid perfume, foundations, eye shadow, lipstick, etc.).

In this respect, the composition of the invention can be in the form of an aerosol or other spray, fragrance diffusers, a wick or other liquid system, or a solid, for instance candles or a wax base as in pomanders and plastics, powders as in sachets or dry sprays or gels, as in solid gel sticks, clothes deodorants as applied by washing machine applications such as in detergents, powders, liquids, whiteners or fabric softeners, fabric refreshers, linen sprays, closet blocks, closet aerosol sprays, or clothes storage areas or in dry cleaning to overcome residual solvent notes on clothes, bathroom accessories such as paper towels, bathroom tissues, sanitary napkins, towelettes, disposable wash cloths, disposable diapers, and diaper pail deodorants, cleansers such as disinfectants and toilet bowl cleaners, cosmetic products such as antiperspirant and deodorants, general body deodorants in the form of powders, aerosols, liquids or solid, or hair care products such as hair sprays, conditioners, rinses, hair colors and dyes, permanent waves, depilatories, hair straighteners, hair groom applications such as pomade, creams and lotions, medicated hair care products containing such ingredients as selenium sulphide, coal tar or salicylates, or shampoos, or foot care products such as foot powders, liquids or colognes, after shaves and body lotions, or soaps and synthetic detergents such as bars, liquids, foams or powders, agricultural and pet care products such as domestic animal and pet care products including deodorants, shampoo or cleaning agents, or animal litter material. In addition, the composition can be used in a fine fragrance.

In some embodiments, the composition can be further encapsulated in a core-shell microcapsule. Encapsulation of active materials is known in the art, see for example US Patent Nos. 2,800,457, 3,870,542, 3,516,941, 3,415,758, 3,041,288, 5,112,688, 6,329,057, and 6,261,483. Wall forming materials include polyurethane, polysiloxanes, polyurea, polyamide, polyimide, polyvinyl alcohol, polyanhydride, polyolefin, polysulfone, polysaccharide, protein, polylactide (PLA), polyglycolide (PGA), polyorthoester, polyphosphazene, silicone, lipid, modified cellulose, gums, polystyrene, and polyesters or combinations of these materials. Other polymeric materials that are functional are ethylene maleic anhydride copolymer, styrene maleic anhydride copolymer, ethylene vinyl acetate copolymer, and lactide glycolide copolymer. Biopolymers that are derived from alginate, chitosan, collagen, dextran, gelatin, and starch can also be used as the encapsulating materials. Additionally, microcapsules can be made via the simple or complex coacervation of gelatin. Preferred encapsulating polymers include those formed from gelatin, urea-formaldehyde, melamine-formaldehyde, isocyanates, silica, or hydrogel-forming polymers.

In some embodiments, the composition of the invention is provided as a spray-dried composition. Suitable methods for spray drying are provided in, *e.g.,* PCT/US2013/060290. Briefly, the practice involves dispersing and dissolving dry carrier materials (e.g., sugar, sugar derivatives, modified starch, proteins, celluloses, salts, dextrins, gums, sugar alcohols, polyols, peptides, acids, carbohydrates or hydrocolloids) in solvent until free of lumps. The composition is then added under constant agitation until a homogeneous mixture is obtained. The emulsion may be further subjected to high shear or homogenized to reduce oil droplet size prior to spray drying. Subsequently, the mixture or emulsion is spray-dried using any suitable spray dryer. For example, a spray dryer with a vertical parallel flow function can be used. The spray dryer should be a system with a dehumidifying and drying function. For example, a spray dryer capable of blowing a high volume of desiccated air with a dew point of less than 5°C is particularly preferable. For a spray dryer with no dehumidifying and drying function, the spray dryer is inevitably arranged with a dry dehumidifier, e.g., a honeycomb-type rotary dehumidifier (e.g., Nichias Corporation or Sweden PROFLUTE Corporation). Suitable spray dryers include the micro mist spray dryer and the hybrid granulator series manufactured by Fujisaki Electric Co., Ltd.; the fluidized spray dryer FSD with internal fluid bed as manufactured by Niro Corporation; the fluid granulation spray dryer and L-8 type spray dryer manufactured by Ogawara (Japan); the DL-21 type and GB-21 type spray dryers manufactured by Yamato Scientific Co., Ltd., and Anhydro Spray Bed Dryer manufactured by SPX Corporation. Once dried, desirably the composition contains from about 0% to about 15% water. Preferably, the composition will have a water activity of 0.1 to 0.6, or more desirably 0.2 to 0.5, and most preferably from 0.2 to 0.4 wherein said levels of dryness can be achieved with or without secondary drying.

### Applications.

The delivery systems of the present invention are well-suited for use, without limitation, in the following products:
a. Household products
   i. Liquid or Powder Laundry Detergents which can use the present invention include those systems described in U.S. Pat. Nos. 5,929,022, 5,916,862, 5,731,278, 5,565,145, 5,470,507, 5,466,802, 5,460,752, 5,458,810, 5,458,809, 5,288,431, 5,194,639, 4,968,451, 4,597,898, 4,561,998, 4,550,862, 4,537,707, 4,537,706, 4,515,705, 4,446,042, and 4,318,818
   ii. Unit Dose Pouches, Tablets and Capsules such as those described in EP 1 431 382 A1, US 2013/0219996 A1, US 2013/0284637 A1, and US 6,492,315. These unit dose formulations can contain high concentrations of a functional material *(e.g.,* 5-100% fabric softening agent or detergent active), fragrance *(e.g.,* 0.5-100%, 0.5-40%, and 0.5-15%), and flavor (*e.g.,* 0.1-100%, 0.1-40%, and 1-20%). They can contain no water to limit the water content as low as less than 30% *(e.g.,* less than 20%, less than 10%, and less than 5%).
   iii. Scent Boosters such as those described in US 7,867,968, US 7,871,976, US 8,333,289, US 2007/0269651 A1, and US2014/0107010 A1.
   iv. Fabric Care Products such as Rinse Conditioners (containing 1 to 30 weight % of a fabric conditioning active), Fabric Liquid Conditioners (containing 1 to 30 weight % of a fabric conditioning active), Tumble Drier Sheets, Fabric Refreshers, Fabric Refresher Sprays, Ironing Liquids, and Fabric Softener Systems such as those described in U.S. Pat. Nos. 6,335,315, 5,674,832, 5,759,990, 5,877,145, 5,574,179, 5,562,849, 5,545,350, 5,545,340, 5,411,671, 5,403,499, 5,288,417, 4,767,547 and 4,424,134
      Liquid fabric softeners/fresheners containing at least one fabric softening agent present, preferably at a concentration of 1 to 30% (e.g., 4 to 20%, 4 to 10%, and 8 to 15%). The ratio between the active material and the fabric softening agent can be 1 : 500 to 1 : 2 (e.g., 1 : 250 to 1 : 4 and 1 : 100 to 1 :8). As an illustration, when the fabric softening agent is 5% by weight of the fabric softener, the active material is 0.01 to 2.5%, preferably 0.02 to 1.25% and more preferably 0.1 to 0.63%. As another example, when the fabric softening agent is 20% by weight of the fabric softener, the active material is 0.04 to 10%, preferably 0.08 to 5% and more preferably 0.4 to 2.5%. The active material is a fragrance, malodor counteractant or mixture thereof. The liquid fabric softener can have 0.15 to 15% of capsules (e.g., 0.5 to 10%, 0.7 to 5%, and 1 to 3%). When including capsules at these levels, the neat oil equivalent (NOE) in the softener is 0.05 to 5% (e.g., 0.15 to 3.2%, 0.25 to 2%, and 0.3 to 1%).
      Suitable fabric softening agents include cationic surfactants. Non-limiting examples are quaternary ammonium compounds such as alkylated quaternary ammonium compounds, ring or cyclic quaternary ammonium compounds, aromatic quaternary ammonium compounds, diquaternary ammonium compounds, alkoxylated quaternary ammonium compounds, amidoamine quaternary ammonium compounds, ester quaternary ammonium compounds, and mixtures thereof. Fabric softening compositions, and components thereof, are generally described in US 2004/0204337 and US 2003/0060390. Suitable softening agents include esterquats such as Rewoquat WE 18 commercially available from Evonik Industries and Stepantex SP-90 commercially available from Stepan Company.
   v. Liquid dish detergents such as those described in U.S. Pat. Nos. 6,069,122 and 5,990,065
   vi. Automatic Dish Detergents such as those described in U.S. Pat. Nos. 6,020,294, 6,017,871, 5,968,881, 5,962,386, 5,939,373, 5,914,307, 5,902,781, 5,705,464, 5,703,034, 5,703,030, 5,679,630, 5,597,936, 5,581,005, 5,559,261, 4,515,705, 5,169,552, and 4,714,562
   vii. All-purpose Cleaners including bucket dilutable cleaners and toilet cleaners
   viii. Bathroom Cleaners
   ix. Bath Tissue
   x. Rug Deodorizers
   xi. Candles
   xii. Room Deodorizers
   xiii. Floor Cleaners
   xiv. Disinfectants
   xv. Window Cleaners
   xvi. Garbage bags/trash can liners
   xvii. Air Fresheners including room deodorizer and car deodorizer, scented candles, sprays, scented oil air freshener, Automatic spray air freshener, and neutralizing gel beads
   xviii. Moisture absorber
   xix. Household Devices such as paper towels and disposable Wipes
   xx. Moth balls/traps/cakes
b. Baby Care Products
   i. Diaper Rash Cream/Balm
   ii. Baby Powder
c.Baby Care Devices
   i. Diapers
   ii. Bibs
   iii. Wipes
d. Oral Care Products. Tooth care products (as an example of preparations according to the invention used for oral care) generally include an abrasive system (abrasive or polishing agent), for example silicic acids, calcium carbonates, calcium phosphates, aluminum oxides and/or hydroxylapatites, surface-active substances, for example sodium lauryl sulfate, sodium lauryl sarcosinate and/or cocamidopropylbetaine, humectants, for example glycerol and/or sorbitol, thickening agents, for example carboxymethyl cellulose, polyethylene glycols, carrageenan and/or Laponite™, sweeteners, for example saccharin, taste correctors for unpleasant taste sensations, taste correctors for further, normally not unpleasant taste sensations, taste-modulating substances (for example inositol phosphate, nucleotides such as guanosine monophosphate, adenosine monophosphate or other substances such as sodium glutamate or 2-phenoxypropionic acid), cooling active ingredients, for example menthol derivatives, (for example L-menthyllactate, L-menthylalkylcarbonates, menthone ketals, menthane carboxylic acid amides), 2,2,2-trialkylacetic acid amides (for example 2,2-diisopropylpropionic acid methyl amide), icilin and icilin derivatives, stabilizers and active ingredients, for example sodium fluoride, sodium monofluorophosphate, tin difluoride, quaternary ammonium fluorides, zinc citrate, zinc sulfate, tin pyrophosphate, tin dichloride, mixtures of various pyrophosphates, triclosan, cetylpyridinium chloride, aluminum lactate, potassium citrate, potassium nitrate, potassium chloride, strontium chloride, hydrogen peroxide, flavorings and/or sodium bicarbonate or taste correctors.
   i. Tooth Paste. An exemplary formulation as follows:
      1. calcium phosphate 40-55%
      2. carboxymethyl cellulose 0.8-1.2%
      3. sodium lauryl sulfate 1.5-2.5%
      4. glycerol 20-30%
      5. saccharin 0.1-0.3%
      6. flavor oil 1.0-2.5%
      7. water q.s. to 100%
         A typical procedure for preparing the formulation includes the steps of (i) mixing by a blender according to the foregoing formulation to provide a toothpaste, and (ii) adding a composition of this invention and blending the resultant mixture till homogeneous.
   ii. Tooth Powder
   iii. Oral Rinse
   iv. Tooth Whiteners
   v. Denture Adhesive
e. Health Care Devices
   i. Dental Floss
   ii. Toothbrushes
   iii. Respirators
   iv. Scented/flavored condoms
f. Feminine Hygiene Products such as Tampons, Feminine Napkins and Wipes, and Pantiliners
g. Personal Care Products: Cosmetic or pharmaceutical preparations, e.g., a "water-in-oil" (W/O) type emulsion, an "oil-in-water" (O/W) type emulsion or as multiple emulsions, for example of the water-in-oil-in-water (W/O/W) type, as a PIT emulsion, a Pickering emulsion, a micro-emulsion or nano-emulsion; and emulsions which are particularly preferred are of the "oil-in-water" (O/W) type or water-in-oil-in-water (W/O/W) type. More specifically,
   i. Personal Cleansers (bar soaps, body washes, and shower gels)
   ii. In-shower conditioner
   iii. Sunscreen ant tattoo color protection (sprays, lotions, and sticks)
   iv. Insect repellants
   v. Hand Sanitizer
   vi. Antiinflammatory balms, ointments, and sprays
   vii. Antibacterial ointments and creams
   viii. Sensates
   ix. Deodorants and Antiperspirants including aerosol and pump spray antiperspirant, stick antiperspirant, roll-on antiperspirant, emulsion spray antiperspirant, clear emulsion stick antiperspirant, soft solid antiperspirant, emulsion roll-on antiperspirant, clear emulsion stick antiperspirant, opaque emulsion stick antiperspirant, clear gel antiperspirant, clear stick deodorant, gel deodorant, spray deodorant, roll-on, and cream deordorant.
   x. Wax-based Deodorant. An exemplary formulation as follows:
      1. Parafin Wax 10-20%
      2. Hydrocarbon Wax 5-10%
      3. White Petrolatum 10-15%
      4. Acetylated Lanolin Alcohol 2-4%
      5. Diisopropyl Adipate 4-8%
      6. Mineral Oil 40-60%
      7. Preservative (as needed)
         The formulation is prepared by (i) mixing the above ingredients, (ii) heating the resultant composition to 75 °C until melted, (iii) with stirring, adding 4% cryogenically ground polymer containing a fragrance while maintaining the temperature 75 °C, and (iv) stirring the resulting mixture in order to ensure a uniform suspension while a composition of this invention is added to the formulation.
   xi. Glycol/Soap Type Deodorant. An exemplary formulation as follows:
      1. Propylene Glycol 60-70%
      2. Sodium Stearate 5-10%
      3. Distilled Water 20-30%
      4. 2,4,4-Trichloro-2'- Hydroxy Diphenyl Ether, manufactured by the Ciba-Geigy Chemical Company and a Trademark of the Ciba-Geigy Chemical Company) 0.01-0.5%
         The ingredients are combined and heated to 75 °C with stirring until the sodium stearate has dissolved. The resulting mixture is cooled to 40 °C followed by addition of a composition of this invention.
   xii. Lotion including body lotion, facial lotion, and hand lotion
   xiii. Body powder and foot powder
   xiv. Toiletries
   xv. Body Spray
   xvi. Shave cream and male grooming products
   xvii. Bath Soak
   xviii. Exfoliating Scrub
h. Personal Care Devices
   i. Facial Tissues
   ii. Cleansing wipes
i. Hair Care Products
   i. Shampoos (liquid and dry powder)
   ii. Hair Conditioners (Rinse-out conditioners, leave-in conditioners, and cleansing conditioners)
   iii. Hair Rinses
   iv. Hair Refreshers
   v. Hair perfumes
   vi. Hair straightening products
   vii. Hair styling products, Hair Fixative and styling aids
   viii. Hair combing creams
   ix. Hair wax
   x. Hair foam, hair gel, nonaerosol pump spray
   xi. Hair Bleaches, Dyes and Colorants
   xii. Perming agents
   xiii. Hair wipes
j. Beauty Care
   i. Fine Fragrance - Alcoholic. Compositions and methods for incorporating fragrance capsules into alcoholic fine fragrances are described in US 4,428,869. Alcoholic fine fragrances may contain the following:
      1. Ethanol (1-99%)
      2. Water (0-99%)
      3. A suspending aid including but not limited to: hydroxypropyl cellulose, ethyl cellulose, silica, microcrystalline cellulose, carrageenan, propylene glycol alginate, methyl cellulose, sodium carboxymethyl cellulose or xanthan gum (0.1-10%)
      4. Optionally an emulsifier or an emollient may be included including but not limited to those listed above
   ii. Solid Perfume
   iii. Lipstick/lip balm
   iv. Make-up cleanser
   v. Skin care cosmetic such as foundation, pack, sunscreen, skin lotion, milky lotion, skin cream, emollients, skin whitening
   vi. Make-up cosmetic including manicure, mascara, eyeliner, eye shadow, liquid foundation, powder foundation, lipstick and cheek rouge
k. Consumer goods packaging such as fragranced cartons, fragranced plastic bottles/boxes
l. Pet care products
   i. Cat litter
   ii. Flea and tick treatment products
   iii. Pet grooming products
   iv. Pet shampoos
   v. Pet toys, treats, and chewables
   vi. Pet training pads
   vii. Pet carriers and crates
m. Confectionaries confectionery, preferably selected from the group consisting of chocolate, chocolate bar products, other products in bar form, fruit gums, hard and soft caramels and chewing gum
   i. Gum
      1. Gum base (natural latex chicle gum, most current chewing gum bases also presently include elastomers, such as polyvinylacetate (PVA), polyethylene, (low or medium molecular weight) polyisobutene (PIB), polybutadiene, isobutene-isoprene copolymers (butyl rubber), polyvinylethylether (PVE), polyvinylbutyether, copolymers of vinyl esters and vinyl ethers, styrene-butadiene copolymers (styrene-butadiene rubber, SBR), or vinyl elastomers, for example based on vinylacetate/vinyllaurate, vinylacetate/vinylstearate or ethylene/vinylacetate, as well as mixtures of the mentioned elastomers, as described for example in EP 0 242 325, U.S. Pat. No. 4,518,615, U.S. Pat. No. 5,093,136, U.S. Pat. No. 5,266,336, U.S. Pat. No. 5,601,858 or U.S. Pat. No. 6,986,709.) 20-25%
      2. Powdered sugar 45-50%
      3. glucose 15-17%
      4. starch syrup 10-13%
      5. plasticizer 0.1 %
      6. flavor 0.8-1.2%
         The components described above were kneaded by a kneader according to the foregoing formulation to provide a chewing gum. Encapsulated Flavor or sensate is then added and blended till homogeneous.
   ii. Breath Fresheners
   iii. Orally Dissolvable Strips
   iv. Chewable Candy
   v. Hard Candy
n. Baked products, preferably selected from the group consisting of bread, dry biscuits, cakes and other cookies;
o. snack foods, preferably selected from the group consisting of baked or fried potato chips or potato dough products, bread dough products and corn or peanut-based extrudates;
   i. Potato, tortilla, vegetable or multigrain chips
   ii. Popcorn
   iii. Pretzels
   iv. Extruded stacks
p. Cereal Products preferably selected from the group consisting of breakfast cereals, muesli bars and precooked finished rice products
q. Alcoholic and non-alcoholic beverages, preferably selected from the group consisting of coffee, tea, wine, beverages containing wine, beer, beverages containing beer, liqueurs, schnapps, brandies, sodas containing fruit, isotonic beverages, soft drinks, nectars, fruit and vegetable juices and fruit or vegetable preparations; instant beverages, preferably selected from the group consisting of instant cocoa beverages, instant tea beverages and instant coffee beverages
   i. Ready to drink liquid drinks
   ii. Liquid Drink Concentrates
   iii. Powder Drinks
   iv. Coffee: Instant Cappucino
      1. Sugar 30-40%
      2. Milk Powder 24-35%
      3. Soluble Coffee 20-25%
      4. Lactose 1-15%
      5. Food Grade Emulsifier 1-3%
      6. Encapsulated Volatile Flavor 0.01-0.5%
   v. Tea
   vi. Alcoholic
r. Spice blends and consumer prepared foods
   i. Powder gravy, sauce mixes
   ii. Condiments
   iii. Fermented Products
s. Ready to heat foods: ready meals and soups, preferably selected from the group consisting of powdered soups, instant soups, precooked soups
   i. Soups
   ii. Sauces
   iii. Stews
   iv. Frozen entrees
t. Dairy Products milk products, preferably selected from the group consisting of milk beverages, ice milk, yogurt, kefir, cream cheese, soft cheese, hard cheese, powdered milk, whey, butter, buttermilk and partially or fully hydrolyzed milk protein-containing products Flavored milk beverages
   i. Yoghurt
   ii. Ice cream
   iii. Bean Curd
   iv. Cheese
u. Soya protein or other soybean fractions, preferably selected from the group consisting of soya milk and products produced therefrom, soya lecithin-containing preparations, fermented products such as tofu or tempeh or products produced therefrom and soy sauces;
v. Meat products, preferably selected from the group consisting of ham, fresh or raw sausage preparations, and seasoned or marinated fresh or salt meat products
w. Eggs or egg products, preferably selected from the group consisting of dried egg, egg white and egg yolk
x. Oil-based products or emulsions thereof, preferably selected from the group consisting of mayonnaise, remoulade, dressings and seasoning preparations
y. fruit preparations, preferably selected from the group consisting of jams, sorbets, fruit sauces and fruit fillings; vegetable preparations, preferably selected from the group consisting of ketchup, sauces, dried vegetables, deep-frozen vegetables, precooked vegetables, vegetables in vinegar and preserved vegetables
z. Flavored pet foods.

The above-listed applications are all well known in the art. For example, fabric softener systems are described in US Patent Nos. 6,335,315, 5,674,832, 5,759,990, 5,877,145, 5,574,179; 5,562,849, 5,545,350, 5,545,340, 5,411,671, 5,403,499, 5,288,417, and 4,767,547, 4,424,134. Liquid laundry detergents include those systems described in U.S. Patent Nos. 5,929,022, 5,916,862, 5,731,278, 5,565,145, 5,470,507, 5,466,802, 5,460,752, 5,458,810, 5,458,809, 5,288,431, 5,194,639, 4,968,451, 4,597,898, 4,561,998, 4,550,862, 4,537,707, 4,537,706, 4,515,705, 4,446,042, and 4,318,818. Liquid dish detergents are described in U.S. Patent Nos. 6,069,122 and 5,990,065. Shampoo and conditioners that can employ the present invention include those described in US Patent Nos. 6,162,423, 5,968,286, 5,935,561, 5,932,203, 5,837,661, 5,776,443, 5,756,436, 5,661,118, 5,618,523, 5,275,755, 5,085,857, 4,673,568, 4,387,090 and 4,705,681. Automatic Dish Detergents are described in U.S. Pat. Nos. 6,020,294, 6,017,871, 5,968,881, 5,962,386, 5,939,373, 5,914,307, 5,902,781, 5,705,464, 5,703,034, 5,703,030, 5,679,630, 5,597,936, 5,581,005, 5,559,261, 4,515,705, 5,169,552, and 4,714,562.

All parts, percentages and proportions refer to herein and in the claims are by weight unless otherwise indicated.

The values and dimensions disclosed herein are not to be understood as being strictly limited to the exact numerical values recited. Instead, unless otherwise specified, each such value is intended to mean both the recited value and a functionally equivalent range surrounding that value. For example, a value disclosed as "50%" is intended to mean "about 50%."

All publications cited herein are incorporated by reference in their entirety.

The following examples are provided as specific embodiments of the present invention.

### EXAMPLE 1: Preparation of JEFFAMINE-Fragrance Adducts

JEFFAMINE ED900 polyetheramine (8.0 g/ 8.9 mmol) was combined with the fragrances listed in Table 1 (2 equiv total to JEFFAMINE) in a vial and the mixture was stirred neat at room temperature for 24 hours. The resulting products (10.4 g) were submitted for NMR analysis.

**TABLE 1**

| Fragrance Ingredient | Fragrance Structure | % Di-imine Product Yield (NMR) | % free fragrance ingredient |
|---|---|---|---|
| 6-Decenal | 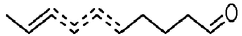 | 97 | 3 |
| 2-octahydro-1H-4,7-methanoinden-5-yl)acetaldehyde | 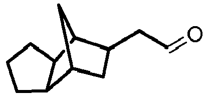 | 89 | 11 |
| 3-(4-isobutylcyclohexyl)pro panal | 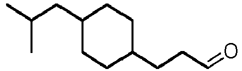 | 93 | 6 |
| mixture (1:1) | 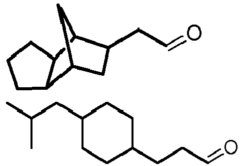 | 93 (mixed adduct) | 7 |
| δ-damascone | 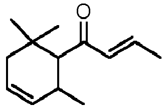 | 80 | 20 |
| methyl acrylate | 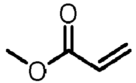 | 50 | 50 |

This analysis indicated that di-imine products were readily formed between the JEFFAMINE and fragrances. Therefore, such materials are of use in a fragrance accord as a delayed delivery system.

### EXAMPLE 2: Malodor Reduction

Imines and Michael adducts are known to hydrolyze slowly in the presence of dilute aqueous acid, which can be from a number of commonly occurring sources. In turn, this triggers the release of the sequestered fragrance ingredient, providing a longer period of fragrance performance.

Malodor acids, such as butyric and isovaleric, can be used as acidic triggers to cause the hydrolysis and release of the fragrance ingredient. The resulting free amine is able to sequester the malodor acid in an acid-base ion pair, reducing the concentration of malodor acid in the headspace (Scheme 3).

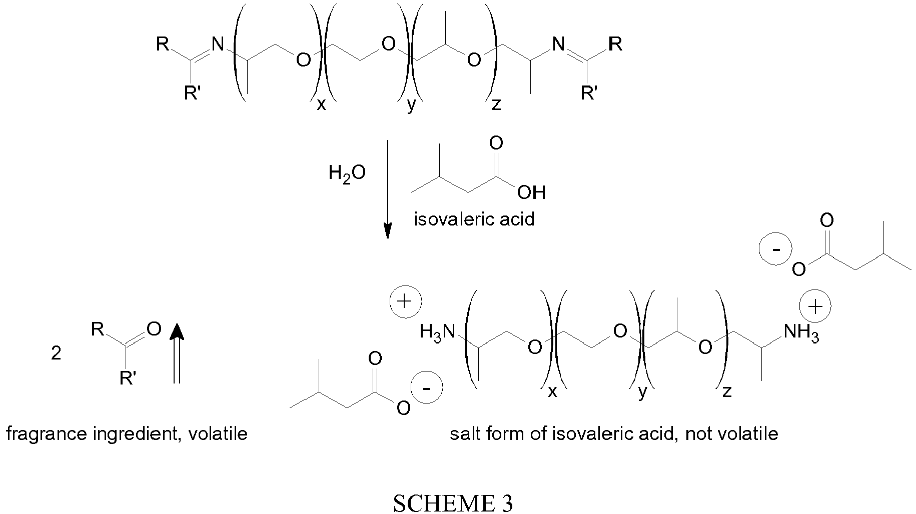

GC Headspace (HS) analysis of JEFFAMINE ED900 and the reactive polymer described in 14/731,865, versus model malodor compounds, isovaleric acid (IVA), *n*-butylamine (*n*BA), and pentanethiol (PT) versus a diethylphthalate control were compared (Table 2).

**TABLE 2**

| Material | % Reduction in HS concentration | | |
|---|---|---|---|
| | IVA | *n*BA | PT |
| JEFFAMINE ED-900 | 99% | 0% | 94% |
| Reactive Polymer | 86% | 100% | 98% |

### EXAMPLE 3: Accords Containing Aldehydes

Aldehyde-rich accords of particular use in the fragrance delivery composition of this invention are listed in Table 3.

**TABLE 3**

| Formula | Ingredient | Parts |
|---|---|---|
| 1 | methyloctyl acetaldehyde | 180 |
| | 3,7-dimethyl-6-octen-1-ol | 30 |
| | 4-(heptyloxy)-3-methylbutanal | 100 |
| | dimethyl benzyl carbinol | 50 |
| | 3,7-dimethyl-1,6-octadien-3-ol | 100 |
| | 4-(4-methyl-3-pentenyl)- 3-cyclohexene-1-carboxaldehyde | 150 |
| | 6-decenal | 20 |
| | phenylacetaldehyde | 20 |
| | 2,6-dimethyloctan-2-ol | 150 |
| 2 | decanal | 90 |
| | 2-methyldecanal | 100 |
| | 2-methylundecanal | 150 |
| | 4-(heptyloxy)-3-methylbutanal | 100 |
| | hexahydro-4,7-methanoindan-1-carboxaldehyde | 25 |
| | petitgrain oil | |
| | sinensal natural 20 ex orange | 10 |
| | 2,6-dimethyloctan-2-ol | 10 |
| | 7-methyl-3-methylene-6-octenal | 300 |
| | | 15 |
| 3 | decanal | 1 |
| | dodecanal | 1 |
| | benzyl acetate | 58 |
| | 3,7-dimethyloct-6-en-1-yl propionate | 10 |
| | 3-(4-isopropylphenyl)-2-methylpropanal | 150 |
| | (e)-3,7-dimethylocta-2,6-dien-1-ol | 225 |
| | (e)-2-benzylideneoctanal | 150 |
| | 4,4a,5,9b-tetrahydroindeno[1,2-d][1,3]dioxine | 10 |
| | 2-((3,7-dimethyloct-6-en-1 -yl)oxy)acetaldehyde | 15 |
| | pheynethyl alcohol | 150 |
| 4 | decanal | 60 |
| | dodecanal | 60 |
| | 2-methylundecanal | 60 |
| | 1-((2-(tert-butyl)cyclohexyl)oxy)butan-2-ol | 70 |
| | 4-(heptyloxy)-3-methylbutanal | 100 |
| | 2,6-dimethyloct-7-en-2-ol | 300 |
| | 2-methyl-4-(2,2,3-trimethylcyclopent-3-en-1-yl)butanal | 150 |
| 5 | benzyl acetate | 70 |
| | 3,7-dimethyl-6-octenal | 10 |
| | 3-(4-isopropylphenyl)propanal | 550 |
| | 4,8-dimethyl-4,9-decadienal | 15 |
| | phenylacetaldehyde | 15 |
| | (2,2-dimethoxyethyl)-benzene | 140 |

## Claims

1. A fragrance adduct that is a reaction product between a polymeric amine and a reactive fragrance ingredient,
wherein
the polymeric amine, having a molecular weight of 100 to 10,000,000 Daltons, contains a backbone and at least one amino functional group,
the reactive fragrance ingredient is an aldehyde, a ketone, an α,β-unsaturated electrophilic compound reactive towards the polymeric amine, or a combination thereof, and
the reaction product is an imine compound or a Michael addition adduct.

2. The fragrance adduct of claim 1, wherein the backbone is polyalkylene or polyoxyalkyleneamine.

3. The fragrance adduct of claim 1, wherein the polymeric amine is polyoxyalkyleneamine containing propylene oxide, ethylene oxide, butylene oxide or a mixture thereof.

4. The fragrance adduct of claim 1, wherein the polymeric amine is 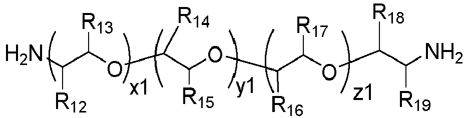 y1 being 1 to 50 and x1 + z1 being 0 to 100
each of R₁₂, R₁₃, R₁₄, R₁₅, R₁₆, R₁₇, R₁₈, and R₁₉, independently, being H,
CH₃, or CH₂CH₃ 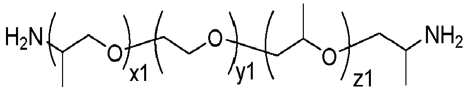
y1 being 1 to 50 and x1 + z1 being 0 to 100 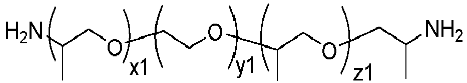 y1 being 1 to 50 and x1 + z1 being 0 to 100 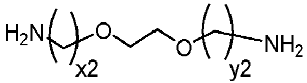 x2 being 0 to 50 and y2 being 0 to 50 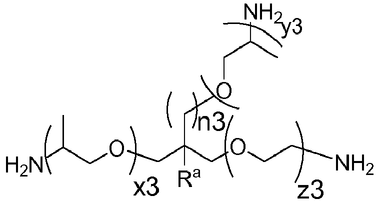 x3+y3+z3 being 1 to 200, n3 being 0 to 10, and R^{a} being H, CH₃, or C₂H₅ 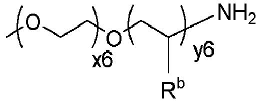 x6 being 1 to 80, y6 being 1 to 80, and R^{b} being H, CH₃, or CH₂CH₃ 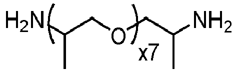 x7 being 1 to 80 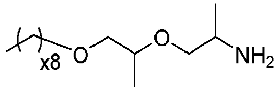 x8 being 4 to 24 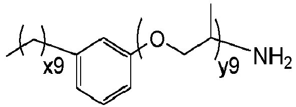 x9 being 1 to 18 and y9 being 5 to 25 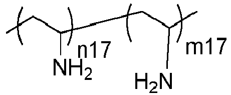 each of m17 and n17, independently, being 300 to 40,000 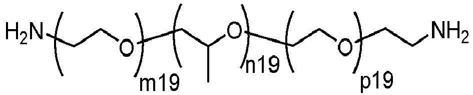 each of m19, n19, and p19, independently, being 1 to 100 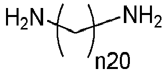 n20 being 6 to 24 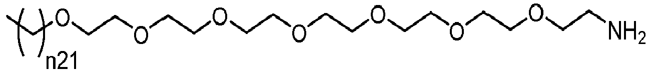 n21 being 10 to 50 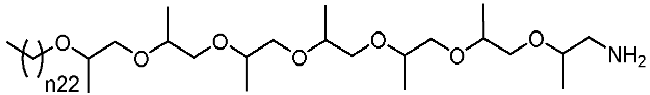 n22 being 14 to 50 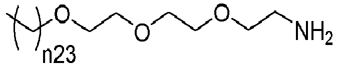 n23 being 10 to 50 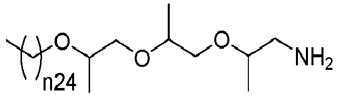 n24 being 10 to 50 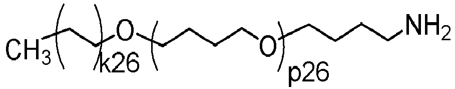 k26 being 4 to 25 and p26 being 2 to 20 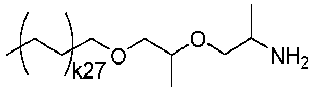 k27 being 2 to 24

5. The fragrance adduct of claim 4, wherein the polymeric amine is 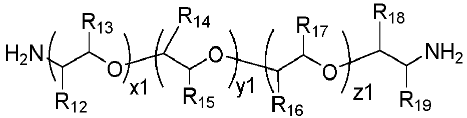 y1 being 3 to 20 and x1 + z1 being 20 to 50
each of R₁₂, R₁₃, R₁₄, R₁₅, R₁₆, R₁₇, R₁₈, and R₁₉, independently, being H,
CH₃, or CH₂CH₃ 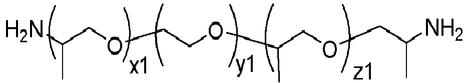
y1 being 3 to 20 and x1 + z1 being 20 to 50 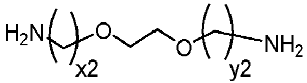 x2 being 2 to 12 and y2 being 2 to 12 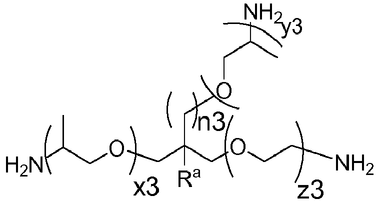 x3+y3+z3 being 50 to 120, n3 being 0 to 4, and R^{a} being H, or CH₃ 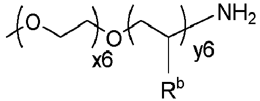 x6 being 4 to 50, y6 being 4 to 50, and R^{b} being H, or CH₃ 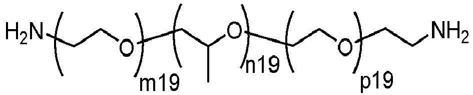 each of m19, n19, and p19, independently, being 4 to 40.

6. The fragrance adduct of claim 1, or of any preceding claim, wherein the polymeric amine has a molecular weight of 200 to 5000 Daltons.

7. The fragrance adduct of any one of claims 1-6, wherein the reactive fragrance ingredient is a aldehyde, a ketone, an α,β-unsaturated carbonyl compound, an α,β-unsaturated nitrile, an α,β-unsaturated nitroxide, or a combination thereof.

8. The fragrance adduct of any one of claims 1-7, wherein the reactive fragrance ingredient is 6-decenal, 3-(4-isobutylcyclohexyl)propanal, 2-octahydro-1H-4,7-methanoinden-5-yl)acetaldehyde, 2-methylpentan-3-yl (E)-but-2-enoate, 4-(heptyloxy)-3-methylbutanal, 7,7,8,9,9-pentamethyl-6,7,8,9-tetrahydro-SH-cyclopenta[h]quinazoline, or a combination thereof.

9. The fragrance adduct of any one of claims 1-8, wherein the reaction product is an imine compound.

10. The fragrance adduct of any one of claims 1-9, wherein the reaction product is a Michael addition adduct.

11. A fragrance composition comprising the fragrance adduct of claim 1 or of any preceding claim.

12. A household product, personal care product, hair care product or beauty care product comprising the fragrance adduct of claim 1 or of any of claims 2 to 10.

13. A method for sequestering a fragrance comprising binding a fragrance to an polymeric amine to form a fragrance adduct thereby sequestering the fragrance, wherein the fragrance is a an aldehyde, a ketone, an α,β-unsaturated electrophilic compound reactive towards the polymeric amine, or a combination thereof.

14. The method of claim 13, further comprising reacting the fragrance adduct with a malodor acid or malodor thiol compound thereby releasing the fragrance from the fragrance adduct.
